# EUROPEAN PATENT APPLICATION

(11) **EP 4 606 888 A1**
(43) Date of publication of application: **27.08.2025**
(21) Application number: 23879858.1
(22) Date of filing: 19.10.2023
(51) Int. Cl.: C12N 5/0735, C12N 5/0793, C12N 5/0797, C12N 5/10

(54) **NOVEL PRODUCTION METHOD FOR NEURAL STEM CELL/NEURAL PROGENITOR CELL**

(30) Priority: 20.10.2022 JP 2022168047
(71) Applicant: K Pharma, Inc., Tokyo 106-0032 (JP)
(72) Inventor: LIN Yu-Ching Zachary, Yokohama-shi, Kanagawa, 240-0024 (JP); TANOKASHIRA Daisuke, Fujisawa-shi, Kanagawa 251-8555 (JP)
(74) Representative: Müller, Christian Stefan Gerd
(86) International application number: PCT/JP2023/037824
(87) International publication number: WO 2024/085215

(57) **Abstract**

Although pluripotent stem cells, once transferring to CTraS, exhibit significant improvement in the efficiency of subsequent cell differentiation, there is a problem of poor reproducibility for stably inducing pluripotent stem cells to CTraS, and consequently is a problem of a low efficiency in the differentiation induction to target neural lineage cells. Additionally, when target neural lineage cells need to be differentiated from pluripotent stem cells in a future, it is necessary to make the differentiation to the neural lineage cells more efficiently. The present inventors have demonstrated the above problem can be solved by providing a novel method capable of inducing pluripotent stem cells into a state in which such cells are easily induced to differentiate to neural lineage cells stably in a future. Specifically, the present inventors showed that the above problems can be solved by adding four small molecules, SB431542, LDN193189, SU5402, and EC23 and culturing pluripotent stem cells for 5 days.

## Description

### Technical Field

The present invention relates to providing a cell culture method for more stably and reliably producing neural stem cells/neural progenitor cells from pluripotent stem cells.

### Background Art

Pluripotent stem cells (e.g., ES cells, iPS cells) are cells with the pluripotency differentiate to various tissues and cells that constitute the body in the process of differentiation, and are considered to be widely applicable in the fields of regenerative medicine and research. For realizing such applications, it is necessary to quickly and efficiently induce differentiation to the desired tissues and cells.

When pluripotent stem cells are cultured in a suspended state, they form spherical cell aggregates, and various intercellular interactions are generated within the aggregates to form an embryoid body (EB) including cells of three germ layers (ectoderm, mesoderm, endoderm). In many of the cell types targeted in clinical studies or researches, the desired cells are obtained from EB by applying an optimal culture method for respective cell types.

However, the pluripotent stem cells are originally have variations from cell to cell, and such variations cause the variations in differentiation, thereby often having often caused the problem of not being able to to obtain the desired cells. To solve such a problem, it has been shown that when three small molecules, SB431542, Dorsomorphin, and CHIR99021, which regulate signals involved with the mechanism of maintaining undifferentiation are added to a medium for pluripotent stem cells in an undifferentiated state, it is possible to transition the pluripotent stem cells to "Chemically Transitional EB-like State" (CTraS), a state where the rate and efficiency of differentiation to the desired cell are significantly improved, in just only 5 days of culture (Patent Literatures 1 to 3, Non Patent Literature 1).

It was shown that, the pluripotent stem cells transitioned to CTraS are mixture of ectoderm-derived cells, mesoderm-derived cells, and endoderm-derived cells, and the rate and efficiency of differentiation to the desired cells are significantly improved regardless of the type of pluripotent stem cell lines from which these cells are derived. For example, even in a pluripotent stem cell line that is difficult to differentiate into the neural cells, cells transitioned to CTraS efficiently differentiate into the desired neural cells, and it was shown that, when compared to a conventional method that required as long as 66 days to form neural cell-aggregated neurospheres (Non Patent Literature 2), it took only 20 days to form neural cell-aggregated neurospheres by transferring to CTraS (Non Patent Literature 3), which significantly improved the efficiency of cell differentiation.

### Citation List

### Patent Literature

Patent Literature 1: WO2017/141900
Patent Literature 2: WO2017/170328
Patent Literature 3: WO2018/043476

### Non Patent Literature

Non Patent Literature 1: Fujimori et al., Stem Cell Reports, 9(5), 1675-1691, 2017
Non Patent Literature 2: Nori et al., PNAS, 108(40) 16825-16830, 2011
Non Patent Literature 3: Fujimori et al., Nat Med., 24(10), 1579-1589, 2018

### Summary of Invention

### Technical Problem

However, although pluripotent stem cells, once transferring to CTraS, exhibit significant improvement in the efficiency of subsequent cell differentiation, there is a problem of poor reproducibility for stably inducing pluripotent stem cells to CTraS, and consequently is a problem of a low efficiency in the differentiation induction to target neural lineage cells. Additionally, when target neural lineage cells need to be differentiated from pluripotent stem cells in a future, it is necessary to make the differentiation to the neural lineage cells more efficiently.

### Solution to Problem

The inventors of the present invention have demonstrated the above problems can be solved by providing a novel method capable of inducing pluripotent stem cells into a state in which such cells are easily induced to differentiate to neural lineage cells stably in a future. Specifically, the present inventors showed that the above problem can be solved by adding four small molecules, SB431542, LDN193189, SU5402, and EC23 and culturing pluripotent stem cells for 5 days.

More specifically, the present application provides the following embodiments to solve the problems described above:
[1]: A method for producing a neural stem cell/a neural progenitor cell from a pluripotent stem cell, comprising: a step of culturing a pluripotent stem cell for 5 days in a medium containing a SMAD signaling pathway inhibitor, retinoic acid (RA) or a RA analog at a concentration of 100 nM or less, and an FGF signaling pathway inhibitor;
[2]: The method according to [1], wherein the neural stem cell/the neural progenitor cell is characterized by the increased expressions of PAX6, SOX1 and NESTIN and by the suppressed expression of OCT4;
[3]: The method according to [1], wherein the pluripotent stem cell is selected from the group consisting of an ES cell and an iPS cell;
[4]: The method according to [2], wherein the pluripotent stem cell is selected from the group consisting of an ES cell and an iPS cell;
[5]: The method according to any of [1] to [4], wherein the SMAD signaling pathway inhibitor is SB431542 and LDN193189;
[6]: The method according to any of [1] to [4], wherein the RA analog is EC23;
[7]: The method according to any of [1] to [4], wherein the FGF signaling pathway inhibitor is SU5402;
[8]: The method according to any of [1] to [4], wherein the medium is selected from the group consisting of a medium for primate ES/iPS cells, AK02N medium, ciKIC (Registered trademark) iPS medium, mTeSR medium, NutriSTEM medium, and Essential 8 medium;
[9]: A method for producing a neurosphere from a pluripotent stem cell, the method comprising the steps of:
   (i) culturing a pluripotent stem cell for 5 days in a medium containing a SMAD signaling pathway inhibitor, RA or a RA analog at a concentration of 100 nM or less, and an FGF signaling pathway inhibitor to produce a neural stem cell/a neural progenitor cell from the pluripotent stem cell, and
   (ii) producing a neurosphere formed from the neural stem cell/the neural progenitor cell;
[10]: The method according to [9], wherein the step (ii) is carried out by culturing for 13 days in the presence of B-27 supplement (Registered trademark), Y27632, LIF, FGF, and RA or a RA analog;
[11] The method according to [10], wherein the RA analog is EC23;
[12]: The method according to any of [9] to [11], wherein the step (ii) is carried out under a hypoxic condition;
[13]: The method according to any of [9] to [11], wherein a Notch signaling inhibitor is further added after 12 days from the start of the culture in the step (ii);
[14]: The method according to [13], wherein the Notch signaling inhibitor is a y(gamma)-secretase inhibitor;
[15]: The method according to any of claims 9 to 11, wherein after the step (ii), a step of dispersing the formed neurosphere into individual cells to produce a neurosphere again is further carried out once or more;
[16]: A method for producing a neural lineage cell from a pluripotent stem cell, the method comprising the steps of:
   (i) culturing a pluripotent stem cell for 5 days in a medium containing a SMAD signaling pathway inhibitor, RA or a RA analog at a concentration of 100 nM or less, and an FGF signaling pathway inhibitor to produce a neural stem cell/a neural progenitor cell from a pluripotent stem cell,
   (ii) producing a neurosphere formed from the neural stem cell/the neural progenitor cell, and
   (iii) producing a neural lineage cell from the neurosphere.

### Advantageous Effects of Invention

By the step of culturing pluripotent stem cells for 5 days in a medium containing a SMAD signaling pathway inhibitor, RA or a RA analog at a concentration of 100 nM or less (low concentration), and an FGF signaling pathway inhibitor using the method for producing neural stem cells/neural progenitor cells of the present invention, it is possible to induce the pluripotent stem cell into neural stem cells/neural progenitor cells stably and with high reproducibility, and to improve more significantly the efficiency of subsequent differentiation into neurospheres and further differentiation into neural lineage cells than conventional methods.

### Brief Description of Drawings

[Figure 1] Figure 1 shows mRNA expression levels of 4 marker factors of PAX6, SOX1, NESTIN, or OCT, when cells were cultured under various culture conditions.
[Figure 2] Figure 2 shows protein expressions of 4 marker factors of PAX6, SOX1, NESTIN, or OCT4, when cells were cultured under various culture conditions.
[Figure 3] Figure 3 shows mRNA expression levels of 4 marker factors of PAX6, SOX1, NESTIN, or OCT4, when cells were cultured under various culture conditions.
[Figure 4] Figure 4 shows mRNA expression levels of 4 marker factors of PAX6, SOX1, NESTIN, or OCT4, when cells were cultured under various culture conditions.
[Figure 5] Figure 5 shows mRNA expression levels of 3 marker factors of PAX6, SOX1, or NESTI, when cells were cultured under various culture conditions.
[Figure 6] Figure 6 shows the fluorescent labeling results of expression of each marker protein when carrying out *in vitro* differentiation induction from PNS.
[Figure 7] Figure 7 shows the fluorescent labeling results of expression of each marker protein when carrying out *in vitro* differentiation induction from SNS.
[Figure 8] Figure 8 shows the result of evaluating walking status of mice to which neurospheres were transplanted on day 9 after spinal cord injury, as BMS score over times up to 9 weeks after spinal cord injury .
[Figure 9] Figure 9 shows the fluorescent labeling results of the expression of various marker proteins in *in vivo* transplanted cells in the mice transplanted with neurospheres.

### Description of Embodiments

It was shown that, prior to the present invention, by culturing undifferentiated pluripotent stem cells in a medium containing 3 small molecules, SB431542, Dorsomorphin, and CHIR99021, which regulate signals involved in the mechanism of undifferentiation maintenance, it was possible to to transition the pluripotent stem cells to "Chemically Transitional EB-like State" (CTraS), the state where the rate and efficiency of differentiation into a target cell were significantly improved, in only 5 days of culture (Non Patent Literature 1).

It was shown that, the pluripotent stem cells transitioned to CTraS are mixture of ectoderm-derived cells, mesoderm-derived cells, and endoderm-derived cells, and the rate and efficiency of differentiation to the desired cells are significantly improved regardless of the type of pluripotent stem cell lines from which these cells are derived, and it was revealed that the pluripotent stem cells transitioned to CTraS exhibit cell characteristics of reduction in OCT4 expression, strong positive PAX6 expression, strong positive SOX1 expression, and strong positive NESTIN expression. Although there was a problem of poor reproducibility for stably inducing pluripotent stem cells to CTraS, and consequently is a problem of a low efficiency in the differentiation induction to target neural lineage cells, the present inventors believed to be able to provide a cell culture method for solving these problems, and continued the study and development.

### Method for producing neural stem cells/neural progenitor cells of the present invention

The inventors of the present invention continued extensive studies and found that by culturing pluripotent stem cells for 5 days in a medium containing a SMAD signaling pathway inhibitor, retinoic acid (RA) or a RA analog at a concentration of 100 nM or less (low concentration), and an FGF signaling pathway inhibitor, it is possible to efficiently induce pluripotent stem cells into neural stem cells/neural progenitor cells, thereby showing that the above problems can be solved.

Thus, the present invention, in an embodiment, provides a method for producing neural stem cells/neural progenitor cells from pluripotent stem cells, comprising a step of culturing pluripotent stem cells for 5 days in a medium containing a SMAD signaling pathway inhibitor, a low concentration retinoic acid (RA) or RA analog, and an FGF signaling pathway inhibitor.

In this method, the SMAD signaling pathway inhibitor, retinoic acid (RA) or the RA analog at a concentration of 100 nM or less (which is lower concentration than the concentration (2 µ(micro)M) used in the general neural differentiation induction), and the FGF signaling pathway inhibitor contained in the medium are contained in the medium at the start of pluripotent stem cell culture (i.e., at Day 0), and the method is characterized by culturing for 5 days in the medium having this composition.

The medium for pluripotent stem cells which can be used in the method for producing neural stem cells/neural progenitor cells of the present invention can be any medium which can be generally used in the art and, for example, Primate ES/iPS Cell Medium, AK02N medium (Ajinomoto Co., Inc.), ciKIC(Registered trademark) iPS Medium (Kanto Chemical Co., Inc.), mTeSR medium (Stem Cell Technologies Inc.), NutriSTEM medium (Sartorius), and Essential 8 medium (Thermo Fisher Scientific Inc.) can be used. Additionally, the method for producing neural stem cells/neural progenitor cells of the present invention aims to culture cells to be transplanted to human, and a serum-free medium is preferably used because it is not desirable to add non-human serum to a medium.

In the case of using these media, coating of the culture plate may be combined for improving the cell culture efficiency, and coating with iMatrix, coating with Matrigel, and coating with Vitronectin can be used as the coating. As examples of the combination of these media and coatings, the combination of AK02N medium and coating with iMatrix, the combination of ciKIC(Registered trademark) iPS Medium and coating with iMatrix, the combination of StemFit medium and coating with iMatrix, the combination of NurtiSTEM medium and coating with Matrigel, the combination of mTeSR medium and coating with Matrigel, and the combination of Essential 8 medium and coating with Vitronectin can be used.

In the present invention, 5 days of the culture period means to culture for 5 days after replacing a medium with the medium containing a SMAD signaling pathway inhibitor, a low concentration retinoic acid (RA) or RA analog, and an FGF signaling pathway inhibitor. The culture period can be changed while observing the conditions of cells. The culture period does not need to be precisely 5 days (that is, 120 hours) and can be changed by around 6 hours.

### Characteristics of raw material cells

The pluripotent stem cells used as the raw material cells in the method for producing neural stem cells/neural progenitor cells of the present invention mean stem cells capable of differentiating into almost all cells that compose the body, and examples include embryonic stem cells (ES cells), embryonic germ cells (EG cells), induced pluripotent stem cells (iPS cells), somatic stem cells, and EG cells. In Japan, ES cells can be supplied as established cell lines from Riken BioResource Research Center, Institute for Life and Medical Sciences of Kyoto University, and the like. Additionally, iPS cells can be supplied as established cell lines from Center for iPS Cell Research and Application of Kyoto University and other institutions. In the present invention, it is preferable to use iPS cells from the viewpoints of ethics and the stability of cell supplies.

Human pluripotent stem cells, particularly iPS cells, have properties of variety in the differentiation efficiency from cell line to cell line and of a relatively slow differentiation rate. For this reason, it is necessary to select pluripotent stem cells capable of easily differentiating to target cells and tissues in advance, and, even if such a selected cell line is used, there is a problem in that intense labors and time are still are required to achieve highly efficient differentiation induction. In contrast, the method for producing neural stem cells/neural progenitor cells from pluripotent stem cells of the present invention makes it possible to produce neural stem cells/neural progenitor cells that are high homogeneous and can be induced to achieve the subsequent differentiation induction in high efficiency.

For example, in the case of using iPS cells in the differentiation induction to neural stem cells, it is known that, in the conventional neurosphere production method, the induction into neural stem cells proceeds comparatively efficiently when the iPS cells are derived are skin cells, whereas the induction into neural stem cells proceeds less efficiently when the iPS cells are derived are blood cells. In contrast, it is shown that, in the method for producing neural stem cells/neural progenitor cells of the present invention, the induction into neural stem cells/neural progenitor cells from pluripotent stem cells efficiently proceeds regardless of the cells from which pluripotent stem cells are derived. Therefore, when iPS cells are used as the raw material cells in the present invention, iPS cells derived from any cells can be used, such as iPS cells derived from blood cells and iPS cells derived from skin cell.

### Characteristics of neural stem cells/neural progenitor cells produced by the present invention

The neural stem cells/the neural progenitor cells produced by the method for producing neural stem cells/neural progenitor cells of the present invention are characterized as neural stem cells/neural progenitor cells which are deemed to proceed to neural lineage cells when compared to the cells produced by the above-mentioned CTraS induction method. Such neural stem cells/neural progenitor cells are characterized by, as the cell markers, the reduction in OCT4 expression, strongly positive PAX6 expression, strongly positive SOX1 expression, and strongly positive NESTIN expression, when compared to pluripotent stem cells as the raw material cells. The reduction in OCT4 expression refers to 50% or less, preferably 20% or less, and more preferably 10% or less, of the OCT4 expression when compared to the OCT4 expression in the raw material iPS cells. The strongly positive PAX6 expression refers to 2 times or more, preferably 5 times or more, and more preferably 10 times or more PAX6 expression, when compared to the PAX6 expression in the raw material iPS cells. The strongly positive SOX1 expression refers to 2 times or more, preferably 5 times or more, and more preferably 10 times or more SOX1 expression, when compared to the SOX1 expression in the raw material iPS cells. The strongly positive NESTIN expression refers to 2 times or more, preferably 5 times or more, and more preferably 10 times or more NESTIN expression, when compared to the NESTIN expression in the raw material iPS cells.

The neural stem cells/the neural progenitor cells produced by the method for producing neural stem cells/neural progenitor cells of the present invention are decisively directed to the subsequent differentiation to neural lineage cells and have significantly improved both maturation and rate of differentiation while maintaining the undifferentiated state. Using such characteristics, these cells can be differentiated efficiently to various neural lineage cells in accordance with the differentiation induction method to various neural lineage cells that have been established in the art.

The neural stem cells/the neural progenitor cells produced by the method for producing neural stem cells/neural progenitor cells of the present invention are defined as the cells having both the self-replication ability which can proliferate while maintaining the undifferentiated state and the multipotency which can differentiate into 3 cell lineages composing the central nervous system, neurons, astrocyte, and oligodendrocyte. The neural stem cells/the neural progenitor cells produced by the method for producing neural stem cells/neural progenitor cells of the present invention, as defined, have the self-proliferation ability, and can form 3 cell lineages: neurons, astrocyte, and oligodendrocyte, when subsequently cultured to differentiate into neural lineage cells, via neural progenitor cell groups, in accordance with differentiation conditions into respective neural lineage cells.

The neural stem cells/the neural progenitor cells produced by the method for producing neural stem cells/neural progenitor cells of the present invention are also characterized in that, as the marker molecules expressed in cells, expressions of PAX6, SOX1, and NESTIN are enhanced and expression of OCT4 is suppressed.

OCT4 is a molecule known as the marker for pluripotent stem cells and is known to be highly expressed in pluripotent stem cells as the raw material. In the neural stem cells/the neural progenitor cells produced by the method for producing neural stem cells/neural progenitor cells of the present invention, the expression of such an OCT4 is reduced, indicating that the neural stem cells/the neural progenitor cells lack the multipotency found in pluripotent stem cells.

On the other hand, the neural stem cells/neural progenitor cells are characterized by gene expressions of the SOX factor group such as SOX1 and SOX2, and gene expression of PAX6. Generally, the efficiency of *in vitro* neural induction is monitored by expression analysis on genes such as NESTIN, PAX6, and the SOX factor group, which are characteristic markers found in neural stem cells/neural progenitor cells (Stem Cells 2005; 23:1234-1241). In line with such findings, it is shown that the neural stem cells/the neural progenitor cells produced by the method for producing neural stem cells/neural progenitor cells of the present invention also exhibit enhanced expressions of PAX6, SOX1, and NESTIN.

### Additives to the culture medium

### (1) SMAD Signaling pathway inhibitor

The method for producing neural stem cells/neural progenitor cells of the present invention is characterized in that a SMAD signaling pathway inhibitor is added to the medium for cells. As the SMAD signaling pathway inhibitor, the method is characterized to use both SB431542 and LDN193189. In accordance with the literature information (Qi et al., Nat Biotechnol. 35(2): 154-163, 2017) describing that the inhibition by SB431542 and DN193189 (this inhibition treatment is referred to as LSB treatment) can cause the highly efficient induction of PAX6-positive neural progenitor cells, the present inventors decided to use dual inhibition of SB431542 and LDN193189 (LSB). The studies in the present description also reveal that the LSB treatment reduces the expression of OCT4 and the expressions of PAX6, SOX1, and NESTIN are conversely enhanced, when compared to pluripotent stem cells as the raw material cell,.

When SB431542 is used as the SMAD signaling pathway inhibitor in the medium for pluripotent stem cells, it is preferable to add the inhibitor in 1 to 10 µ(micro)M, preferably 2 to 5 µ(micro)M, and more preferably 3 µ(micro)M. When LDN193189 is used as the SMAD signaling pathway inhibitor, it is preferable to add the inhibitor in 50 to 250 nM, preferably 100 to 200 nM, and more preferably 150 nM.

In the present invention, the SMAD signaling pathway inhibitor refers to a group of molecules characterized by inhibiting the signaling from TGF β(beta) in a cell and, for example, in addition to SB431542 and LDN193189 described above, compounds such as Noggin and Dorsomorphin are known. In the present invention, any of these can be used in addition to SB431542 and LDN193189, or in place of SB431542 or LDN193189, as long as the expression of OCT4 is reduced and the expressions of PAX6, SOX1, and NESTIN are conversely enhanced, when compared to pluripotent stem cells as the raw material cells.

### (2) Wnt agonist

In the conventional CTraS induction method (Non Patent Literature 1), Wnt agonist CHIR99021 was added to a medium for the cell culture. However, in that method, the expression of SOX10, often found in differentiated neural crest cells, was found to have been enhanced, and it was considered that the addition of CHIR99021 was not preferable for maintaining neural stem cells/neural progenitor cells while maintaining the undifferentiated state. Therefore, it was decided not to add the Wnt agonist CHIR99021 during the cell culture by the method for producing neural stem cells/neural progenitor cells of the present invention.

### (3) Retinoic acid (RA) or RA analog

Retinoic acid (RA) or an RA analog was not used in the conventional CTraS induction method. A previous report (Okada Y. et al, Stem Cells, 2008) showed that PAX6-positive and NESTIN-positive neural progenitor cells were induced by RA at a concentration of 100 nM or less (relatively lower concentration than the concentration (2 µ(micro)M) used in the general neural differentiation induction). Based on this finding, the use of RA or a RA analog was examined in the present invention, and it was confirmed that when RA is added at a concentration of 100 nM or less, the expression level of PAX6 and expression level of NESTIN increase, and when a RA analog, EC23 (Maltman et al., Mol. BioSyst., 5, 458-471, 2009), was added at a concentration of 100 nM or less, the expression level of PAX6 and the expression level of RA increased about twice as much as RA. Considering these results, it was decided to add either RA a a concentration of 100 nM or less, or a RA analog at a concentration of 100 nM or less, to the cell medium. In the preferred embodiment, in the step of producing neural stem cells/neural progenitor cells of the present invention, EC23 as a RA analog is used.

When RA is used in the medium for pluripotent stem cells, it is preferable to be added in a range from 1 nM to 100 nM, preferably 5 nM to 100 nM, and more preferably 10 nM to 100 nM. Similarly, when EC23 is used as a RA analog, it is preferable to be added in a range from 1 nM to 100 nM, preferably 5 nM to 100 nM, and more preferably 10 nM to 100 nM.

In the present invention, RA or a RA analog refers to retinoic acid or a natural or artificial analog thereof, which is a group of molecules that demonstrate the action via binding to a retinoic acid receptor (RAR), and compounds such as AM580, TTNPB, and Adapalene are known to be included in the group in addition to RA and EC23 described above. In the present invention, any of these can be used in addition to retinoic acid (RA) or EC23, or in place of RA or EC23, as long as it reduces the expression of OCT4 and enhances the expressions of PAX6, SOX1, and NESTIN, when compared to pluripotent stem cells as the raw material cells.

### (4) FGF Signaling pathway inhibitor

An FGF signaling pathway inhibitor was not used in the conventional CTraS induction method. Since the FGF signaling has the function for maintaining the stem cell properties of pluripotent stem cells, it is considered that, when pluripotent stem cells are induced to neural stem cells, the use of the FGF signaling inhibitor would facilitate the induction into neural stem cells. Then, the use of an FGF signaling pathway inhibitor was investigated in the present invention, and it was confirmed that when SU5402 was added, the expression level of PAX6 and the expression level of NESTIN increase.

When SU5402 is used as the FGF signaling pathway inhibitor in the medium for pluripotent stem cells, it is preferable to be added in a range from 200 nM to 50 µ(micro)M, preferably 500 nM to 20 µ(micro)M, and more preferably 1 µ(micro)M to 10 µ(micro)M. In a particularly preferable embodiment, 5 µ(micro)M of SU5402 can be used as the FGF signaling pathway inhibitor.

In the present invention, when referring to the FGF signaling pathway inhibitor, an FGF signaling pathway inhibitor that functions by the same mechanism as SU5402 can be used other than SU5402. Examples of such an FGF signaling pathway inhibitor to be used in the present invention include compounds such as Ponatinib (AP24534), Infigratinib (BGJ398), PD173074, Dovitinib (TKI-258), AZD4547, Danusertib (PHA-739358), Lenvatinib (E7080), Lucitanib (E3810)hydrochloride, Sulfatinib, Pemigatinib (INCB054828), ODM-203, Futibatinib (TAS-120), ASP5878, Derazantinib (ARQ-087), Lenvatinib (E7080) Mesylate, PRN1371, PD-166866 (PD166866), S49076, ON123300, FIIN-2, Dovitinib (TKI258) Lactate, Zoligratinib (Debio-1347), LY2874455.

As a result of the above investigations (1) to (4) on the additives to the medium, the present inventors found that, in the present invention, when pluripotent stem cells are cultured in the medium containing (1) a SMAD signaling pathway inhibitor, (3) RA or a RA analog at a concentration of 100 nM or less, and (4) an FGF signaling pathway inhibitor, the expression of OCT4 is reduced in cells, and the expressions of PAX6, SOX1, and NESTIN are increased, thereby showing that pluripotent stem cell-derived neural stem cells/neural progenitor cells can be reproducibly maintained in an undifferentiated state (CTraS) when compared to the conventional CTraS induction method, and can be more efficiently differentiate into neurons when neurons need to be differentiated from pluripotent stem cells in the future.

### Other culture conditions

When pluripotent stem cells are cultured to produce neural stem cells/neural progenitor cells in the present invention, the neural stem cells/neural progenitor cells can be produced by culturing pluripotent stem cells without using feeder cells. When culturing pluripotent stem cells, a method is known in which feeder cells are cultured into a sheet form on a cell culture substrate, on which pluripotent stem cells are cultured. However, since feeder cells often use cells derived from an animal other than human such as mouse, when considering the use of a regenerative medical product manufactured from pluripotent stem cells to human body, it is preferable not to use feeder cells to avoid the risk of immune response. The present invention can provide a method which can produce neural stem cells/neural progenitor cells from pluripotent stem cells using the medium and the additives described earlier without using feeder cells.

### Method for producing neurospheres from pluripotent stem cells

In the present invention, neurospheres can be produced by manufacturing neural stem cells/neural progenitor cells in accordance with the method for producing neural stem cells/neural progenitor cells from pluripotent stem cells described earlier (this step is also referred to as Step (i)), followed by the culture of Step (ii) to be described below to produce neurospheres.

When producing neurospheres from pluripotent stem cells in the present invention, neurospheres can be produced by carrying out specifically the following steps:
(i) a step of culturing pluripotent stem cells for 5 days in a medium containing a SMAD signaling pathway inhibitor, retinoic acid (RA) or a RA analog at a concentration of 100 nM or less, and an FGF signaling pathway inhibitor to produce neural stem cells/neural progenitor cells from pluripotent stem cells, and then
(ii) a step of producing neurospheres from the obtained neural stem cells/neural progenitor cells.

In the above Step (ii), the method for producing neurospheres from neural stem cells/neural progenitor cells can be any method known in the art.

The step of producing neurospheres from the obtained neural stem cells/neural progenitor cells (Step (ii)) can be carried out using a culture method known in the art, such as, for example, the methods described in the scientific papers by Okada et al. (Okada Y. et al, Stem Cells, 2008, 0293), and by Matsumoto et al. (Matsumoto et al., Stem Cell Reports, 6, 422-435, 2016). Specifically, for example, as described in known literatures (Matsumoto et al., Stem Cell Reports, 6, 422-435, 2016, and the like), the neurosphere can be produced by promoting the differentiation from undifferentiated pluripotent stem cells, CTraS-mediated, to the ectoderm by culturing in a hypoxic state, or by culturing neural stem cells/neural progenitor cells in a sphere medium (Hormone mix or KBM medium) supplemented with B-27 supplement(Registered trademark), bFGF, a ROCK inhibitor (e.g., Y27632), hLIF, and a high concentration RA or RA analog (e.g., EC23) for 13 days.

B-27 Supplement(Registered trademark) supplemented to the medium herein refers to a serum-free medium supplement for neuronal cell culture, and composed of 20 factors including insulin (components not disclosed). B-27 Supplement(Registered trademark) is added for the purpose of culturing neural lineage cells for an extended period of time in the step of producing neurospheres. B-27 Supplement(Registered trademark), when used in the medium in the step of producing neurospheres, can be used in the concentration recommended by the provider of this product to the amount of medium and, for example, it is preferable to add in 2% to a medium amount.

The ROCK (Rho-associated protein kinase) inhibitor added to the medium is a substance capable of suppressing the phenomenon which causes apoptosis at cell dispersion in cell manipulation. Examples of the ROCK inhibitor that can be used in the present invention include, but not limited to, Y27632, Fasudil, H-1152, and Wf-536. The ROCK inhibitor, is added in the step of producing neurospheres for the purpose of inducing the differentiation into neurons from neural stem cells/neural progenitor cells.

As an example, it is known that Y27632 that can be used as the ROCK inhibitor is an intense ROCK inhibitor that acts specifically and ATP antagonistically as a potent inhibitor against Ca2+ receptor agonists at myosin phosphorylation and smooth muscle contraction, inhibits cell spreading, and suppresses stress fiber formation in hepatic stellate cells by Rho-A. When Y27632 is used in the medium in the step of producing neurospheres, it is preferable to be added in a range from 500 nM to 50 µ(micro)M, preferably 1 µ(micro)M to 30 µ(micro)M, and more preferably 5 µ(microM to 15 µ(micro)M. In a particularly preferable embodiment, 10 µ(micro)M of Y27632 can be used as the ROCK inhibitor.

The hLIF added to the medium means human LIF (Leukemia Inhibitory Factor). LIF is a cytokine found as a factor to inhibit the growth of leukemia cells to induce the differentiation to macrophages, belongs to the IL-6 family, and activates JAK1 and JAK2 via LIFR/gp 130 receptor. LIF, known to have various functions, is expressed also in neurons and is known to have the function of extending neurons. In the step of producing neurospheres, the hLIF is added for the purpose of inducing the differentiation to neurons from neural stem cells/neural progenitor cells. When the hLIF is used in the medium in the step of producing neurospheres, it is preferable to be added in a range from 500 pg/mL to 50 ng/mL, preferably 1 ng/mL to 30 ng/mL, and more preferably 5 ng/mL to 15 ng/mL. In a particularly preferable embodiment, 10 ng/mL of human LIF can be used.

In relation to the addition of RA or a RA analog in the step of producing neurospheres, the previous report (Okada Y. et al, Stem Cells, 2008) shows that a relatively high concentration of RA, about 10 µ(micro)M, induces more β(beta)III-tubulin-positive postmitotic neurons. Based on this finding, RA or a RA analog, in the step of producing neurospheres, is added for the purpose of inducing the differentiation to neurons from neural stem cells/neural progenitor cells. In this step, a preferable embodiment uses EC23 as the RA analog.

In the step of producing neurospheres, RA or an RA analog is used. For example, when EC23 as the RA analog is used, it is preferable to be added in a range from 1 µ(micro)M to 50 µ(micro)M, preferably 2 µ(micro)M to 30 µ(micro)M, and more preferably 2 µ(micro)M to 10 µ(micro)M.

The Step (ii) of the present invention can be carried out under a hypoxic condition throughout the entire culture period of 13 days. By carrying out the culture under a hypoxic condition in Step (ii), neurospheres can be efficiently produced.

In the Step (ii) of the present invention, after 12 days from the start of the culture period of 13 days, that is, for the last 1 day (24 hours before collecting and freezing neurospheres), a Notch signaling inhibitor can be added. The Notch signaling pathway is the gene regulatory (signal transduction) pathway related to the process of differentiation into various tissues such as nerves, hematopoiesis, blood vessels, and somite and plays an extremely important role , in the cell-to-cell information transmission, and in the gene control associated with the maintenance of development and homeostasis and cell differentiation. In the transplantation using cells induced from pluripotent stem cells, since highly undifferentiated cells remained in the transplanted cells after transplantation may become tumorigenic, it is considered important to prevent this tumorigenicity and to establish safety. The tumorigenicity can be suppressed by treating pluripotent stem cell-derived neural stem cells/neural progenitor cells using a drug (e.g., y(gamma)-secretase inhibitor) which inhibits the Notch signal associated deeply with multipotency and self-proliferation ability in neural stem cells, thereby removing highly undifferentiated cells or transplanting more differentiated cells. The Notch signaling inhibitor refers to a group of drugs which have an action for inhibiting any of the Notch signaling pathways.

Particularly, in the Notch signaling pathway, a part of y(gamma)-secretase complex was cleaved such information is transferred to the cell nucleus to transcribe and express a target gene. The y(gamma)-secretase inhibitor is known to inhibit this cleavage to function as the Notch signaling pathway inhibitor and to promote the differentiation into various tissues such as nerves. Thus, a y(gamma)-secretase inhibitor can be used as the Notch signaling inhibitor. Examples of the y(gamma)-secretase inhibitor that can be used in the method of the present invention include DAPT and Compound 34, and it is preferable to use DAPT.

When DAPT, y(gamma)-secretase inhibitor, is used as the Notch signaling inhibitor in the medium for pluripotent stem cells, it is preferable to be added in a range from 1 µ(micro)M to 20 µ(micro)M, preferably 5 µ(micro)M to 15 µ(micro)M, and more preferably 7 µ(micro)M to 13 µ(micro)M, and, for example, 10 µ(micro)M can be used.

In the present invention, when neurospheres are obtained in the Step (ii) as described above, the neurospheres can be directly used for the subsequent application, or neurospheres which are obtained by a further step of dissociating the neurospheres into individual cells, and then performing the same step as the Step (ii) described above once or more times to obtain neurospheres, can be used for the subsequent application. In the case of carrying out the additional step to produce neurospheres once, the obtained neurospheres may be obtain to as secondary neurospheres, and in the case of carrying out such a step twice, the obtained neurospheres may be called as as tertiary neurospheres. Thus, the name of neurospheres will differ depending on the number of additional steps carried out.

The neurospheres produced by this method can be used for transplantation for the purpose of regeneration of nervous tissues, such as transplantation to an injury site of spinal cord injury, and transplantation to an injury site of cerebral infarction. The cells of the transplanted neurosphere proliferate in the tissues to which the neurosphere cells are transplanted and also differentiate into neural lineage cells appropriate to the transplanted site by the action of the tissue site to which the neurosphere cells are transplanted.

### Method for producing neural lineage cells from pluripotent stem cells

The present invention also provides a method for producing neural lineage cells from pluripotent stem cells via the method for producing neurospheres via neural stem cells/neural progenitor cells from pluripotent stem cells described earlier.

The term "Neural lineage cells" refers to any of 3 cell lineages of neurons, astrocytes and oligodendrocytes, and any of 3 cell lineages can be differentiate from neural stem cells/neural progenitor cells produced by the method of the present invention by culturing under respective differentiation induction conditions.

In the present invention, when neural lineage cells are produced from pluripotent stem cells, neural lineage cells can be specifically produced by;
(i) a step of culturing pluripotent stem cells for 5 days in a medium containing a SMAD signaling pathway inhibitor, retinoic acid (RA) or a RA analog at a concentration of 100 nM or less, and an FGF signaling pathway inhibitor to produce neural stem cells/neural progenitor cells from pluripotent stem cells, in accordance with the method for producing neural stem cells/neural progenitor cells described earlier,
(ii) a step of producing neurospheres formed from the obtained neural stem cells/the neural progenitor cells, and
(iii) a step of producing neural lineage cells from the neurospheres.

For example, after producing neural stem cells/neural progenitor cells by the method of the present invention (Step (i)), then using the obtained neural stem cells/neural progenitor cells to produce neurospheres by the method described in Non Patent Literature 1 (Step (ii)), 5 x 10³ cells of neurosphere-derived cells obtained in the previous step are further seeded in a 96-well multiplate (falcon) and cultured for 6 weeks in a sphere medium (also referred to as Hormone mix or KBM medium) to which 2% B-27 supplement(Registered trademark) was added, and EC23 is added in place of RA, whereby efficiently inducing maturation of the differentiation of neurospheres into neurons (Step (iii)).

The step of producing neural lineage cells from the neurospheres (Step (iii)) can also be carried out using the culture method known in the art. Specifically, the culture of neurospheres produced by Step (ii) described above can be performed by adding 2% B-27 supplement(Registered trademark) to medium MHM (Media Hormone Mix) for neural stem cells and culturing for 5 to 40 days.

In the present invention, when neurospheres are obtained in the Step (ii) as described above, the neurospheres can be directly used for the Step (iii), or the neurospheres can be used for the subsequent the Step (iii) after carrying out a step of producing neurospheres once or more times which comprise a step of dispersing the neurospheres produced in Step (ii) into individual cells, and a step of subsequently producing neurospheres again by the same step as Step (ii) described above.

The differentiation into neurons can be confirmed based on the enhanced expression of neuronal markers such as TUJ1 and MAP2, and the reduced expression of neural stem cell markers such as SOX1 and PAX6.

When neurospheres are induced to differentiate in accordance with Step (iii), the neurospheres differentiate into 3 neural lineage cells: neurons, astrocytes, and oligodendrocytes.

Hereinafter, the present invention will be specifically described in reference to examples. The following examples do not intend in any way to limit the present invention.

### Examples

### Example 1: Examination on culture conditions when producing neural stem cells/neural progenitor cells from pluripotent stem cells (1)

The present example was carried out for the purpose of examining culture conditions when producing neural stem cells/neural progenitor cells from pluripotent stem cells.

That is, additives that can be used to more efficiently produce neural stem cells/neural progenitor cells from pluripotent stem cells were examined in the present example. Specifically, based on SB431542 (Tocris Bioscience) and Dorsomorphin, which are "SMAD signaling pathway inhibitors", and CHIR99021 (ReproCELL), which is a "Wnt agonist", that have been used to produce neural stem cells/neural progenitor cells from pluripotent stem cells (Non Patent Literature 1), by changing the composition of these, culture conditions were searched under which neural stem cells/neural progenitor cells were more efficiently produced.

First, Dorsomorphin, that had been used in a medium in the conventional method, was decided not to use as the additive since it was considered to be toxic.

On the other hand, since "SMAD signaling pathway inhibitor", as previously published (Chambers S.M. et al, Nat Biotechnol., 30 (7), 715 to 720 (2012)), is reported that inhibition of 2 SMAD signaling pathways, i.e., BMP signaling pathway and TGFβ(beta) signaling pathway, in human ES cells, can differentiate 80% or more cells into neurons under adherent culture conditions, the present inventors decided to investigate the use of LDN193189 (StemRD) in addition to SB431542 (Tocris Bioscience). In this case, 3 µ(micro)M of SB431542 (Tocris Bioscience) and 150 nM of LDN193189 (StemRD) were added to the medium. These amounts were determined based on the description in a known literature (Chambers, SM., et al., 2012 Nat. Biotechnol., 30(7), 715-720).

Additionally, CHIR99021 (ReproCELL) used in the conventional method (Non Patent Literature 1) was used as the "Wnt agonist". In this case, 3 µ(micro)M of CHIR99021 (ReproCELL) was added to the medium.

The previous report (Okada Y. et al, Stem Cells, 2008) showed that a relatively low concentration of retinoic acid (RA) of about 10 nM induces PAX6-positive and NESTIN-positive neural progenitor cells, which are the indicators of neural stem cells/neural progenitor cells. Based on this finding, in this example, the present inventors decided to add "retinoic acid (RA) or a RA analog" in addition to the LSB treatment and to examine the use of EC23 (Cayman) as the "RA analog" (LSBE treatment) . In this case, 10 nM of EC23 (Cayman) was added to the medium.

Based on the above studies, the culture was carried out as follows in the present example. For the raw material cells, iPS cells (ReproCELL, RCRP002N), which are pluripotent stem cells, were used. The cells were seeded at a cell density of 1.5 x 10⁴ cells per well on a cell culture substrate (6-well plate) on which iMatrix-511 silk (MATRIXOME Inc.) (2.4 µ(micro)g/ml) was coated and passage cultured in AK02N medium (Ajinomoto Co., Inc.) for 1 week while basically changing the medium every day. Seventy to 80% confluent iPS cells was treated with 0.5 x TrypLE select (Gibco) and seeded at a cell density of 3 x 10⁴ cells per well, as in the above, on a 6-well plate on which iMatrix-511 silk (MATRIXOME Inc.) was coated. The AK02N medium (Ajinomoto Co., Inc.) contains 10 µ(micro)M of a Rock inhibitor (Y27632, CultureSURE). After seeding, the 6-well plate was incubated overnight under the conditions of 37°C and 5% CO₂, and this point of time was defined as Day 0.

On Day 1, after observing whether the cells adhere well to the plate, the medium was changed to 3 ml of a medium (AK02N-based medium containing 3 (microM of SB431542 (Tocris Bioscience) and 150 nM of LDN193189 (StemRD)), which were incubated overnight under the conditions of 37°C and 5% CO₂.

The cells were cultured up to Day 6 with changing the medium on Day 2 and Day 5 to 3 ml of a medium containing the following additives, to carry out an attempt to produce neural stem cells/neural progenitor cells from the raw cells:
· "LSB": the condition of culture in AK02N-based medium containing 150 nM of LDN193189 (StemRD) and 3 µ(micro)M of SB431542 (Tocris Bioscience) as 2 kinds of "SMAD signaling pathway inhibitor" (hereinafter referred to as LSB treatment);
· "LSBC": the condition of culture in AK02N-based medium further containing 3 µ(micro)M of CHIR99021 (ReproCELL) as the "Wnt agonist", in addition to 150 nM of LDN193189 (StemRD) and 3 µ(micro)M of SB431542 (Tocris Bioscience) (hereinafter, culturing under this culture condition is referred to as LSBC treatment);
· "LSBE": the condition of culture in AK02N-based medium further containing 10 nM of EC23 (Cayman) as the "retinoic acid (RA) or a RA analog", in addition to 150 nM of LDN193189 (StemRD) and 3 µ(micro)M of SB431542(Tocris Bioscience) (hereinafter, culturing under this culture condition is referred to as LSBE treatment);
· "iPSC" is the condition of culture in AK02N-based medium free of additives.

In the present example, the production of neural stem cells/neural progenitor cells was investigated for the indicators of characteristics of neural stem cells/neural progenitor cells of reduced expression of OCT4 and conversely increased expressions of PAX6, SOX1, and NESTIN, when compared to pluripotent stem cells (iPS cells), which are the raw material cells.

In the present example, the expressions of 4 factors, PAX6, SOX1, NESTIN, and OCT4, were examined for the expression level of nucleic acid and the expression level of protein, respectively.

The expression level of nucleic acid was confirmed by extracting mRNA from the gene of each factor, constructing cDNA, and carrying out quantitative PCR using known primers for detecting the sequence thereof, thereby quantifying a mRNA expression level of each gene.

The mRNA extraction was carried out using a RNeasy plus micro kit (Qiagen). Each cell sample of 1 x 10⁵ cells or less was dissolved in 350 µ(micro)l of Buffer RLT plus + 3.5 µ(micro)l of β(beta)-ME and mRNA is extracted in accordance with the product's manual, followed by the cDNA synthesis. For the cDNA synthesis, 8 µ(micro)l of the mRNA sample was added into a PCR tube with 2 µ(micro)l of SuperScript VILO (Thermo Fischer) and thoroughly mixed. Then, the sample mixture was allowed to stand in the PCR apparatus under the condition of 25°C for 10 minutes, reacted at 42°C for 1 hour, reacted at 85°C for 5 minutes, and stored at 4°C. After cDNA synthesis, the concentration of each cDNA sample was diluted to 5 ng/µ(micro)l.

For the quantitative PCR, 5 µ(micro)l of Thunderbird Sybr mix (TOYOBO), 0.3 µ(micro)l of 10 µ(micro)M forward primer, 0.3 µ(micro)l of 10 µ(micro)M reverse primer, 0.2 µ(micro)l of 50X ROX, 3.2 µ(micro)l of water and 1 µ(micro)l of 5 ng/µ(micro)l cDNA template were mixed to obtain each reactant. Each sample was prepared in triplicate and added to a 384-well plate which were subjected to a real time PCR system ViAA7 to analyze each sample. The primer set for each factor was as follows:
PAX6: 5'-accacaccggtttcctccttcaca-3' (SEQ ID NO: 1)
   5'-ttgccatggtgaagctgggcat-3' (SEQ ID NO: 2)
SOX1: 5'-gatcagcaagcgcctggggg-3' (SEQ ID NO: 3)
   5'-agcagcgtcttggtcttgcgg-3' (SEQ ID NO: 4)
NESTIN: 5'-ttccctcagctttcaggaccccaa-3' (SEQ ID NO: 5)
   5'-aaggctggcacaggtgtctcaa-3' (SEQ ID NO: 6)
OCT4: 5'-ttgggctcgagaaggatgtggt-3' (SEQ ID NO: 7)
5'-tgcatagtcgctgcttgatcgc-3' (SEQ ID NO: 8)

The protein expression level was confirmed by using the antibody against protein of each factor to visualize a protein expression level of each gene by fluorescent labeling.

Each cell was washed with PBS 3 times, and each sample was fixed with 70 µ(micro)l of 4% PFA for 15 minutes. Then, the cells were washed with PBS 3 times and permeabilized with 0.1% triton-x for 15 minutes. The cells were washed with PBS 3 times and blocked overnight with 3% BSA/PBS at 4°C. On the second day, the cells were washed with PBS 3 times, to which primary antibodies (anti-OCT4 antibody (Millipore), anti-PAX6 antibody (Abcam), anti-SOX1 antibody (Abcam), anti-NESTIN antibody (Millipore), anti-TUJ1 (Beta-Tubulin III) antibody (Sigma), anti-MAP2 antibody (Sigma), anti-OLIG2 antibody (R&D), or anti-GFAP antibody (Synaptic system)) in 1% BSA/PBS were added , and then stored at 4°C overnight. Herein, OCT4 is an iPS cell marker; PAX6, SOX1, and NESTIN are neural stem cell markers; TUJ1 and MAP2 are neuronal markers; OLIG2 is an oligodendrocyte marker; and GFAP is an astrocyte marker. On the third day, the cells were washed with PBS 3 times, to which secondary antibodies (donkey anti-rabbit IgG (Alexa Flour 555), donkey anti-mouse IgG (Alex Flour 488), donkey anti-goat IgG (Alex Flour 555), goat anti-rat IgG (Alex Flour 4888), goat anti-guinea pig IgG (Alex Flour 555), Hoechst 33342 (all Thermo Fisher Scientific Inc.)) and DAPI in 1% BSA/PBS were added, and reacted in a dark place at room temperature for 1 hour. The cells were washed 3 times and observed using a fluorescence microscope (Keyence).

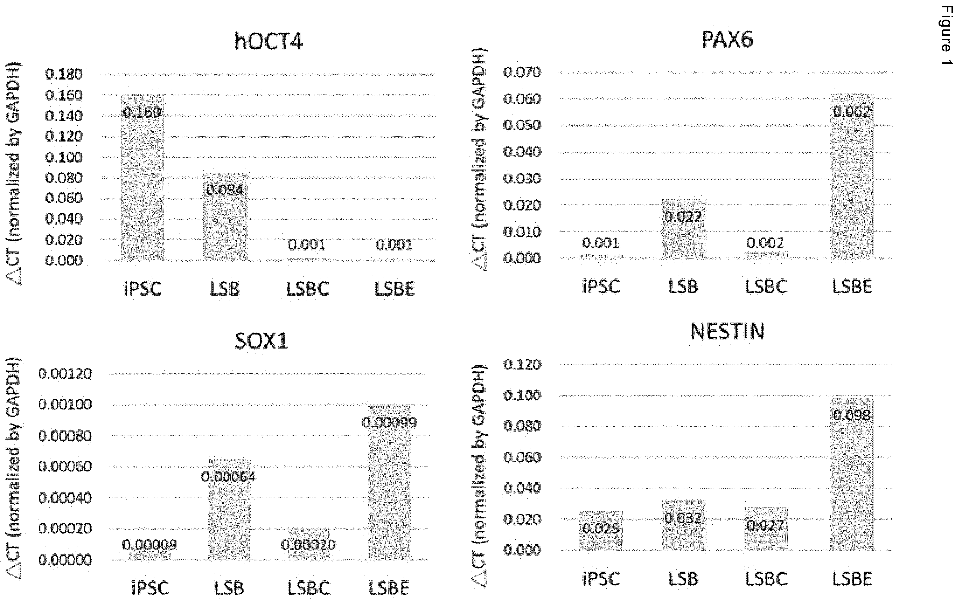

The results on expression levels of mRNA are shown in Figure 1. In the figure, the followings are respectively shown:
· "LSB": shows the mRNA expression level of each factor when iPS cells were treated with LSB condition;
· "LSBC": shows the mRNA expression level of each factor when iPS cells were treated with LSBC condition;
· "LSBE": shows the mRNA expression level of each factor when iPS cells were treated with LSBE condition;
· "iPSC" shows the mRNA expression level of each factor when iPS cells were cultured without additives.

In this figure, when culture conditions were examined for each factor that satisfied the criteria of reduced expression of OCT4 and increased expressions of PAX6, SOX1, and NESTIN when compared to pluripotent stem cells (iPS cells) as the raw material cells, the followings were shown;
· Reduced OCT4 expression: Compared to "iPSC", LSB treatment, LSBC treatment, or LSBE treatment is preferable, with LSBC treatment and LSBE treatment being more preferable;
· Increased PAX6 expression: Compared to "iPSC", LSB treatment or LSBE treatment is preferable, with LSBE treatment being more preferable;
· Increased SOX1 expression: Compared to "iPSC", LSB treatment or LSBE treatment is preferable, with LSBE treatment being more preferable;
· Increased NESTIN expression: Compared to "iPSC", LSB treatment or LSBE treatment is preferable, with LSBE treatment being more preferable.

These findings showed that, in the present invention, the method for culturing in the medium to which CHIR99021 (ReproCELL) as the Wnt agonist was added (LSBC treatment) is preferable from the viewpoint of reducing the expression of OCT4, but this is not preferable from the viewpoint of increasing the expressions of PAX6, SOX1, and NESTIN.

On the other hand, it was shown that the method of culturing in the medium to which LDN193189 (StemRD)and SB43154 (Tocris Bioscience) as SMAD signaling pathway inhibitors were added (LSB treatment) and the method of culturing in the medium to which EC23 (Cayman) as the RA analog was further added to LSB treatment (LSBE treatment) are preferable methods from the viewpoint of reducing the expression of OCT4 and increasing the expressions of PAX6, SOX1, and NESTIN, and LSBE treatment is more preferable method. These results showed that cells having preferable marker phenotypes of neural stem cells/neural progenitor cells are obtained by culturing 5-days.

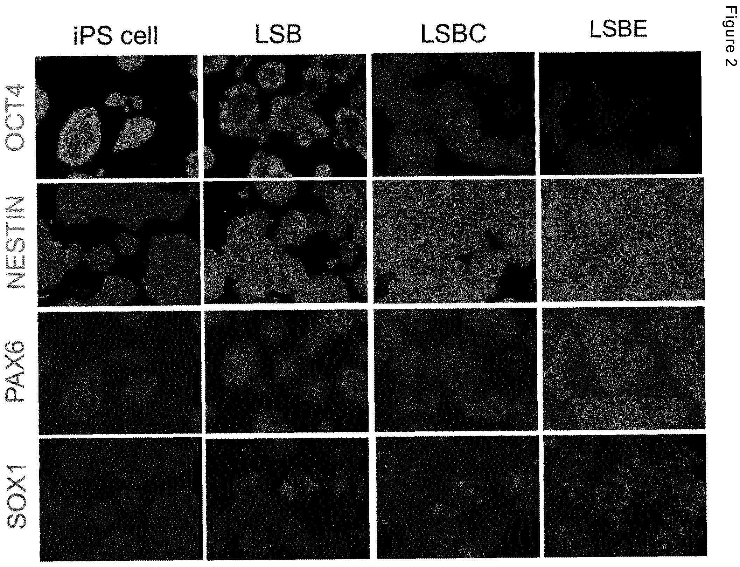

The results on protein expression of each factor are shown in Figure 2. It was shown that these results on protein expressions are correlated with the results on expression levels of mRNA of each factor in Figure 1 described earlier.

### Example 2: Examination on culture conditions when producing neural stem cells/neural progenitor cells from pluripotent stem cells (2)

The present example was carried out for the purpose of further examining culture conditions when producing neural stem cells/neural progenitor cells from pluripotent stem cells.

In the present example, the concentration of EC23 (Cayman) was determined as the RA analog, which was decided to be added to the medium as the result of Example 1, compared to retinoic acid (RA) at a relatively low concentration of about 10 nM reported in the previous report (Okada Y. et al, Stem Cells, 2008). In this example, 10 nM of retinoic acid (RA) (Sigma-Aldrich) or 10 nM of EC23 (Cayman) were added to the medium. These amounts are the values determined based on the fact that the sufficient effect was found even in 10 nM shown in the study conducted in advance on the concentrations from 10 nM to 100 nM.

Based on the above examinations, in the present example, iPS cells (ReproCELL, RCRP002N), which are pluripotent stem cells, were used as in Example 1 as the raw material cells, and cultured under the same conditions as in Example 1 up to Day 1.

The cells were cultured up to Day 6 with changing the medium on Day 2 and Day 5 to 3 ml of a medium containing the following additives, to carry out an attempt to produce neural stem cells/neural progenitor cells from the raw cells:
· "LSBC": the condition of culture in AK02N-based medium further containing 3 µ(micro)M of CHIR99021 (ReproCELL) as the "Wnt agonist", in addition to 150 nM of LDN193189 (StemRD) and 3 µ(micro)M of SB431542 (Tocris Bioscience) (hereinafter, culturing under this culture condition is referred to as LSBC treatment);
· "LSBE": the condition of culture in AK02N-based medium further containing 10 nM of EC23 (Cayman) as the "retinoic acid (RA) or RA analog", in addition to 150 nM of LDN193189 (StemRD) and 3 µ(micro)M of SB431542 (Tocris Bioscience) (hereinafter, culturing under this culture condition is referred to as LSBE treatment);
· "LSBRA": the condition of culture in AK02N-based medium further containing 10 nM of retinoic acid (RA) as the "retinoic acid (RA) or a RA analog", in addition to 150 nM of LDN193189 (StemRD) and 3 µ(micro)M of SB431542 (Tocris Bioscience) (hereinafter, culturing under this culture condition is referred to as LSBRA treatment);
· "iPSC" is the condition of culture in AK02N-based medium free of additives.

Also in the present example, as in Example 1, the production of neural stem cells/neural progenitor cells was investigated for the indicators of characteristics of neural stem cells/neural progenitor cells of reduced expression of OCT4 and conversely increased expressions of PAX6, SOX1, and NESTIN, when compared to pluripotent stem cells (iPS cells) as the raw material cells.

Since it was confirmed in Example 1 that, in the expressions of 4 factors, PAX6, SOX1, NESTIN, and OCT4, the expression level of nucleic acid and the expression level of protein are correlated, in the present example, the expressions of 4 factors, PAX6, SOX1, NESTIN, and OCT4 were investigated for the expression level of nucleic acid. The expression level of nucleic was confirmed by the same method as the method of Example 1.

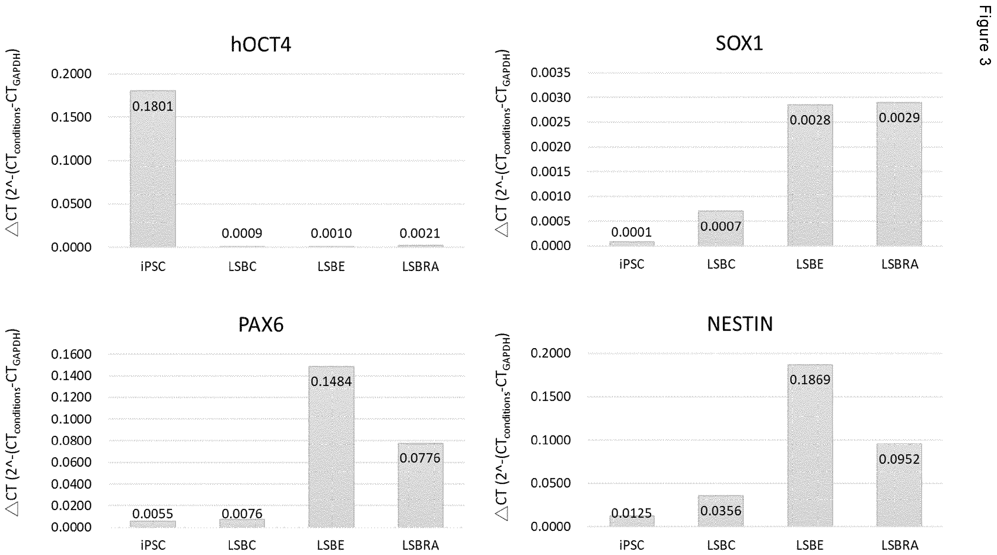

The results on expression levels of mRNA are shown in Figure 3. In the figure, the followings are respectively shown:
· "LSBC" shows the expression level of each factor when iPS cells were treated with LSBC condition;
· "LSBE" shows the expression level of each factor when iPS cells were treated with LSBE condition;
· "LSBRA" shows the expression level of each factor when iPS cells were treated with LSBRA condition;
· "iPS" shows the expression level of each factor when iPS cells were cultured without additives.

When culture conditions were examined that satisfied the criteria of reduced expression of OCT4 and increased expressions of PAX6, SOX1, and NESTIN, when compared to pluripotent stem cells (iPS cells) as the raw material cells, as the characteristics of neural stem cells/neural progenitor cells, the followings were shown;
· LSBC treatment, LSBE treatment, or LSBRA treatment is preferable for reducing OCT4 expression;
· LSBE treatment or LSBRA treatment is preferable for increasing PAX6 expression, with LSBE treatment being more preferable;
· LSBC treatment, LSBE treatment, or LSBRA treatment is preferable for increasing SOX1 expression, with LSBE treatment and LSBRA treatment being more preferable;
· LSBE treatment and LSBRA treatment are preferable for increasing NESTIN expression, with LSBE treatment being more preferable.

These findings showed that, in the present invention, when adding "retinoic acid (RA) or a RA analog", it is more preferable to use EC23 (Cayman) as the RA analog (LSBE treatment). As a result, it was shown that cells having more preferable marker phenotypes of neural stem cells/neural progenitor cells can be obtained by culturing for 5-days.

### Example 3: Examination on culture conditions when producing neural stem cells/neural progenitor cells from pluripotent stem cells (3)

The present example was carried out for the purpose of further examining culture conditions when producing neural stem cells/neural progenitor cells from pluripotent stem cells.

In the present example, the effect of adding SU5402 to the medium was confirmed based on the findings reported in the previous report (Qi Y. et al, Nat Biotechnol., 2017) that the differentiation into neurons was promoted by addition of SU5402 (Cayman) as the FGF signaling pathway inhibitor. In this example, 5 µ(micro)M of SU5402(Cayman) was added to the medium.

Based on the above investigations, in the present example, iPS cells (ReproCELL, RCRP002N), which are pluripotent stem cells, were used as the raw material cells, as in Example 1, and were cultured under the same conditions as in Example 1 up to Day 1.

The cells were cultured up to Day 6 with changing the medium on Day 2 and Day 5 to 3 ml of a medium containing the following additives, to carry out an attempt to produce neural stem cells/neural progenitor cells from the raw material cells:
· "LSBC": the condition of culture in AK02N-based medium further containing 3 µ(micro)M of CHIR99021 (ReproCELL) as the "Wnt agonist", in addition to 150 nM of LDN193189 (StemRD) and 3 µ(micro)M of SB431542 (Tocris Bioscience) (hereinafter, culturing under this culture condition is referred to as LSBC treatment);
· "LSBE": the condition of culture in AK02N-based medium further containing 10 nM of EC23 (Cayman) as the "retinoic acid (RA) or a RA analog", in addition to 150 nM of LDN193189 (StemRD) and 3 µ(micro)M of SB431542 (Tocris Bioscience) (hereinafter, culturing under this culture condition is referred to as LSBE treatment);
· "LSBES": the condition of culture in AK02N-based medium further containing 5 (micro)M of SU5402 (Cayman) as the FGF signaling pathway inhibitor, in addition to 150 nM of LDN193189 (StemRD), 3 µ(micro)M of SB431542 (Tocris Bioscience), and 10 nM of EC23 (Cayman) (hereinafter, culturing under this culture condition is referred to as LSBES treatment);
· "iPSC" is the condition of culture in AK02N-based medium free of additives.

Also in the present example, as in Example 1, the production of neural stem cells/neural progenitor cells was investigated for the indicators of characteristics of neural stem cells/neural progenitor cells of reduced expression of OCT4 and conversely increased expressions of PAX6, SOX1, and NESTIN, when compared to pluripotent stem cells (iPS cells) as the raw material cells.

Additionally, as in Example 2, also in the present example, the expressions of 4 factors of PAX6, SOX1, NESTIN, and OCT4 were confirmed by the same method as the method of Example 1.

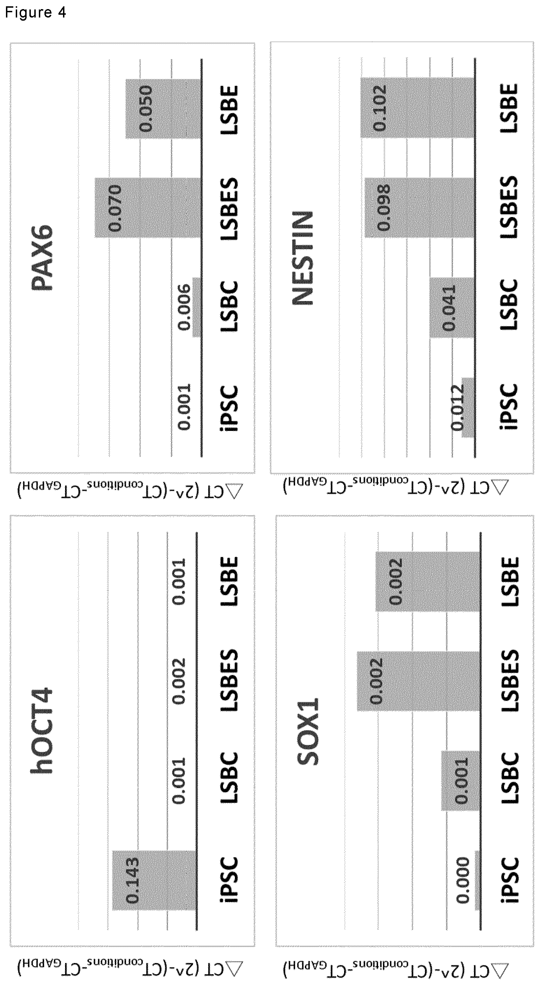

The results on expression levels of mRNA are shown in Figure 4. In the figure, the followings are respectively shown;
· "LSBC" shows the expression level of each factor when iPS cells were treated with LSBC condition;
· "LSBE" shows the expression level of each factor when iPS cells were treated with LSBE condition;
· "LSBES" shows the expression level of each factor when iPS cells were treated with LSBES condition;
· "iPSC" shows the expression level of each factor when iPS cells were cultured without additives.

When culture conditions were examined that satisfied the criteria of reduced expression of OCT4 and increased expressions of PAX6, SOX1, and NESTIN, when compared to pluripotent stem cells (iPS cells) as the raw material cells, as the characteristics of neural stem cells/neural progenitor cells, the followings were shown;
· LSBC treatment, LSBE treatment, or LSBRA treatment is preferable for reducing OCT4 expression;
· LSBE treatment or LSBES treatment is preferable for increasing PAX6 expression, with LSBES treatment being more preferable;
· LSBC treatment, LSBE treatment or LSBES treatment is preferable for increasing SOX1 expression, with LSBE treatment and LSBES treatment being more preferable, and LSBES treatment being further preferable;
· LSBC treatment, LSBE treatment or LSBES treatment is preferable for increasing NESTIN expression, with LSBE treatment and LSBES treatment being more preferable.

The results of Example 1 to Example 3 revealed that, for producing neural stem cells/neural progenitor cells using iPS cells as raw material cells, it is preferable to use either of LSB treatment, LSBE treatment, LSBRA treatment, or LSBES treatment, more preferably LSBE treatment or LSBES treatment, and even more preferably LSBES treatment. As a result, it was shown that cells having further preferable marker phenotypes of neural stem cells/neural progenitor cells can be obtained by culturing for 5-days.

### Example 4: Formation of neurospheres (1)

The present example was carried out for the purpose of examining the step of producing neurospheres using the manufactured neural stem cells/neural progenitor cells as a result of examining in Examples 1 to 3.

In the present example, the neural stem cells/the neural progenitor cells manufactured by LSBES treatment, which was confirmed as the most preferable method in Example 3, were used as raw material cells for neurosphere production.

The neural stem cells/the neural progenitor cells were seeded in a low cell adhesion PrimeSurface 96-well multiplate (Sumitomo Bakelite Co., Ltd.) at a cell density of 1.6 x 10² cells per well. The medium used was PNS medium (i.e., KBM medium containing 2% B-27 supplement (Registered trademark), 20 ng/mL of bFGF (Peprotech), 10 ng/mL of hLIF (Nacalai Tesque Inc.), 10 µ(micro)M of Y27632 (CultureSURE), 3 µ(micro)M of CHIR99021 (ReproCELL), 2 µ(micro)M of SB431542 (Tocris Bioscience) and 2 µ(micro)M of EC23 (Cayman)), and a half of the medium was replaced every other day. When the medium was replaced, cells that did not form cell aggregates were removed.

After 5 days of cell culture, the culture products (primary neurospheres, PNS) were passaged and seeded in a 96-well multiplate (falcon) at a cell density of 4.2 x 10² cells per well. The medium used was SNS medium (i.e., KBM medium containing 2% B-27 supplement, 20 ng/mL of bFGF (Peprotech), 10 ng/mL of hLIF (Nacalai Tesque Inc.), 10 µ(micro)M of Y27632 (CultureSURE), 2 µ(micro)M of SB431542 (Tocris Bioscience) and 2 µ(micro)M of EC23 (Cayman)), and a half of the medium was replaced every other day, and then the cells were cultured for 7 more days (SNS), for a total of 12 days.

### Example 5: Formation of neurospheres (2)

The present example was carried out for the purpose of further examining the step of manufacturing neurospheres examined in Example 4.

In the present example, as in Example 4, the neural stem cells/the neural progenitor cells manufactured by LSBES treatment, which was confirmed as the most preferable method in Example 3, were used as raw material cells for the neurosphere manufacture.

LSBES-treated iPS cells cultured up to Day 6 and reached to a state of almost 90% confluent in Example 3 were washed twice with 2 ml of PBS, then 1 ml of 0.5 x TrypLE select (Gibco) was added to incubate at 37°C for 5 minutes to detach the cells. Five minutes later, the cells were detached using P1000 Pipetman and then transferred to a 15 ml tube containing 9 ml of AK02N medium (Ajinomoto Co., Inc.) (supplemented with Y27632 (CultureSURE)). The cells were centrifuged at 800 rpm for 5 minutes, the supernatant was aspirated, and then 10 ml of AK02N medium (Ajinomoto Co., Inc.) + Y27632 (CultureSURE) was further added to remove TrypLE select (Gibco) (ES group).

After aspirating the supernatant, the pellet was resuspended in 1 ml of AK02N medium (Ajinomoto Co., Inc.) (supplemented with Y27632 (CultureSURE)), thereby counting the cell number.

Then, the cells were seeded in a low cell adhesion PrimeSurface 96-well multiplate (Sumitomo Bakelite Co., Ltd.) in a cell density of 1.6 x 10² cells per well. The medium used was PNS medium (i.e., KBM medium containing 2% B-27 supplement(Registered trademark), 20 ng/mL of bFGF (Peprotech), 10 ng/mL of hLIF (Nacalai Tesque Inc.), 10 µ(micro)M of Y27632 (CultureSURE), 3 µ(micro)M of CHIR99021 (ReproCELL), 2 µ(micro)M of SB431542 (Tocris Bioscience) and 2 µ(micro)M of EC23 (Cayman)), and a half of the medium was replaced every other day, and the culture was carried out under a hypoxic condition for 6 days (up to Day 12). (In the present example, this timing is defined as Day 6). For comparison, the cells of LSBES group were cultured under conditions where EC23 (Cayman) was not added in PNS medium (shown as LSBES PNS-E group).

In this culture, unlike Example 4, an attempt was made to increase the expressions of markers of neural stem cells/neural progenitor cells in all the cells by culturing together all the cells including the cells removed during changing the medium in Example 4.

In the present example, whether the expressions level of PAX6, SOX1 and NESTIN are increased was confirmed by examining their mRNA expression levels using the same method as in Example 1.

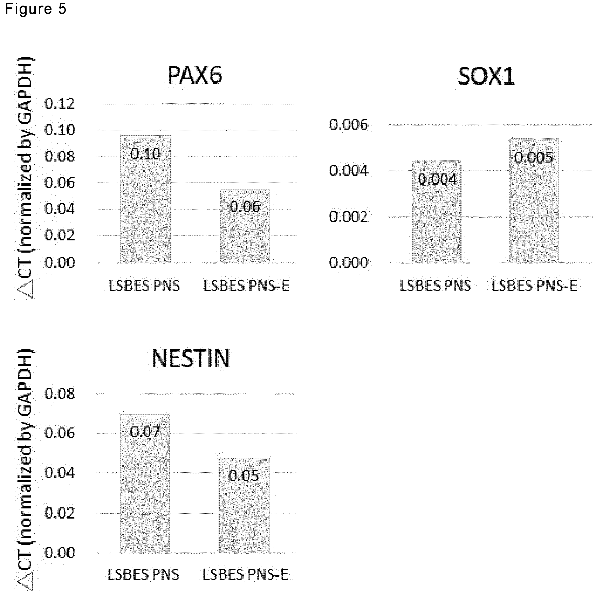

The results on expression levels of mRNA are shown in Figure 5. In Figure 5, the followings are respectively shown;
· "LSBES PNS" shows expression level of each factor in the primary neurospheres (PNS) formed by culturing LSBES-treated neural stem cells/neural progenitor cells in the PNS medium;
· "LSBES PNS-E" shows expression level of each factor in the primary neurospheres (PNS) formed by culturing LSBES-treated neural stem cells/neural progenitor cells in the PNS medium without EC23 (Cayman).

When the expressions of PAX6, SOX1, and NESTIN, which are the markers of neural stem cells/neural progenitor cells, in the cells composing neurospheres were examined, the followings were shown;
· the addition of EC23 (Cayman) to the medium increased PAX6 expression level;
· the addition of EC23 (Cayman) to the medium slightly reduced SOX1 expression but substantially remained unchanged;
· the addition of EC23 (Cayman) to the medium increased NESTIN expression level.

These findings suggested that, even during the neurosphere formation, the addition of EC23 (Cayman) to the medium can further promote the induction to neural stem cells.

### Example 6: Formation of secondary neurospheres

The present example was carried out for the purpose of confirming that PNS produced in Example 4 or 5 and SNS to be used for actual transplantation in the present invention have the same properties.

On Day 12 of the neurosphere production procedure of Example 5, the PNS was collected, washed twice with PBS, then 1 to 3 ml of 0.5 x TrypLE select (Gibco) was added to incubate at 37°C for 5 minutes to detach spheres. After detachment, TrypLE select (Gibco) was removed by washing twice using the KBM medium with Y27632 (CultureSURE). Then, the detached PNS cells were counted for the cell number and seeded in a low cell adhesion PrimeSurface 96-well multiplate (Sumitomo Bakelite Co., Ltd.) at a cell density of 4.3 x 10² cells per well. The medium used was 100 µ(micro)l of SNS medium (i.e., KBM medium containing 2% B-27 supplement (Registered trademark), 20 ng/mL of bFGF (Peprotech), 10 ng/mL of hLIF (Nacalai Tesque Inc.), 10 µ(micro)M of Y27632 (CultureSURE), 2 µ(micro)M of SB431542 (Tocris Bioscience) and 2 µ(micro)M of EC23 (Cayman)), and a half of the medium was replaced every other day, and the culture was carried out for 7 days. On 7 days after seeding the cells to the plate (i.e., Day 19), the SNS was subjected to a further experiment.

### Example 7: Production of neural lineage cells in vitro

The present example was carried out for the purpose of producing neural lineage cells using the primary neurospheres (PNS) formed in Example 4 or 5 and the secondary neurospheres (SNS) formed in Example 6.

That is, the present example used the PNS produced by LSBES PNS treatment, which was confirmed as the most preferable method in Example 5, the SNS produced in Example 6 using the PNS, and the cells induced to differentiate *in vitro* (ivN) by the following method using the SNS.

Specifically, the SNS was collected, washed twice with PBS, then 1 to 3 ml of 0.5 x TrypLE select (Gibco) was added to incubate at 37°C for 5 minutes to detach spheres. After detachment, TrypLE select (Gibco) was removed by washing twice using the KBM medium with Y27632 (CultureSURE). Then, the obtained single cells were counted for the cell number and seeded in a 96-well multiplate (falcon) at a cell density of 5 x 10³ to 7.5 x 10³ cells per well (in the present example, this timing was defined as Day 0). The medium used was 150 µ(micro)l of KBM medium containing 10 µ(micro)M of Y27632 (CultureSURE), and on Day 1, the medium was replaced to the KBM medium containing 2% B-27 supplement (Registered trademark) but without vitamin A, and then the culture was carried out up to Day 33 while replacing the medium every other day.

In the present example, in addition to OCT4, which is an iPS cell marker, and PAX6, SOX1 and NESTIN, which are neural stem cell/neural progenitor cell markers, whether the expressions of SOX17, which is an endoderm cell marker, T, which is a mesoderm marker, TUJ1 and MAP2, which are neuronal cell markers, OLIG2 and NG2, which are oligodendrocyte markers, and CD44 which is an astrocyte marker were increased/reduced were confirmed by the same method as the method of Example 1. The primer sets for SOX17, T, TUJ1, MAP2, OLIG2, NG2, and CD44 were as follows:
SOX17: 5'-acgctttcatggtgtgggctaag-3' (SEQ ID NO: 9)
   5'-gtcagcgccttccacgacttg-3' (SEQ ID NO: 10)
T: 5'-ccttcagcaaagtcaagctcacc-3' (SEQ ID NO: 11)
   5'-tgaactgggtctcagggaagca-3' (SEQ ID NO: 12)
TUJ1: 5'-atttcatctttggtcagagtggggc-3' (SEQ ID NO: 13)
   5'-tgcaggcagtcgcagttttcac-3' (SEQ ID NO: 14)
MAP2: 5'-ggatcaacggagagctgac-3' (SEQ ID NO: 15)
   5'-tcaggactgctacagcctca-3' (SEQ ID NO: 16)
OLIG2: 5'-ggcgcgcaactacatcct-3' (SEQ ID NO: 17)
   5'-cgctcaccagtcgcttcat-3' (SEQ ID NO: 18)
NG2: 5'-ttgtcctgatggctaatgcct-3' (SEQ ID NO: 19)
   5'-tgggctgctcgatggtgta-3' (SEQ ID NO: 20)
CD44: 5'-tggcacccgetatgtegag-3' (SEQ ID NO: 21)
5'-gtagcagggattctgtctg-3' (SEQ ID NO: 22)

According to the procedure of the present example, in the raw material iPS cells (iPSC), the primary neurospheres (PNS), the secondary neurospheres (SNS), and cells induced to differentiate *in vitro* (ivN), the expression levels of mRNA of the following factors are respectively shown in Table 1;
· OCT4, which is the iPS cell marker,
· PAX6, SOX1 and NESTIN, which are neural stem cell markers,
· SOX17, which is an endoderm marker,
· T, which is a mesoderm marker,
· TUJ1 and MAP2, which are neuronal cell markers,
· OLIG2 and NG2, which are oligodendrocyte markers,
· CD44, which is an astrocyte marker.

As a result of producing neurospheres using the differentiation induction method of the present invention, it was shown that, when compared to iPS cells, the expression levels of iPS cell marker OCT4 in PNS and SNS was significantly reduced, whereas the expression levels of neural stem cell markers PAX6, SOX1, and NESTIN were increased. On the other hand, it was also shown that the expression levels of endoderm marker SOX17 and mesoderm marker T did not increase at all. These results suggest that the specific differentiation into neural stem cells is induced.

Next, as a result of dispersing the SNS and carrying out differentiation induction *in vitro* (ivN), it was shown that, when compared to SNS, the expression levels of PAX6 and SOX1 out of the neural stem cell markers were reduced in ivN, the expression levels of neuronal cell markers TUJ1 and MAP2 were increased in ivN, and the expression levels of oligodendrocyte markers OLIG2 and NG2, and astrocyte marker CD44 were also increased in ivN.

These results suggested that induction of differentiation of neural stem cell-derived neurospheres *in vitro* leads to differentiate to 3 cell lineages of neurons, glial cells, oligodendrocytes.

**[Table 1]**

| | OCT4 | PAX6 | SOX1 | NESTIN |
|---|---|---|---|---|
| iPSC | 0.281 | 0.000 | 0.000 | 0.009 |
| PNS | 0.000 | 0.050 | 0.001 | 0.030 |
| SNS | 0.001 | 0.048 | 0.004 | 0.050 |
| ivN | 0.001 | 0.013 | 0.001 | 0.062 |

| | SOX17 | T | TUJ1 | MAP2 |
|---|---|---|---|---|
| iPSC | 0.000 | 0.000 | 0.023 | 0.016 |
| PNS | 0.000 | 0.000 | 0.096 | 0.011 |
| SNS | 0.000 | 0.000 | 0.306 | 0.101 |
| ivN | 0.000 | 0.000 | 0.458 | 0.692 |

| | OLIG2 | NG2 | CD44 | |
|---|---|---|---|---|
| iPSC | 0.000 | 0.000 | 0.002 | |
| PNS | 0.000 | 0.000 | 0.000 | |
| SNS | 0.000 | 0.000 | 0.000 | |
| ivN | 0.001 | 0.001 | 0.045 | |

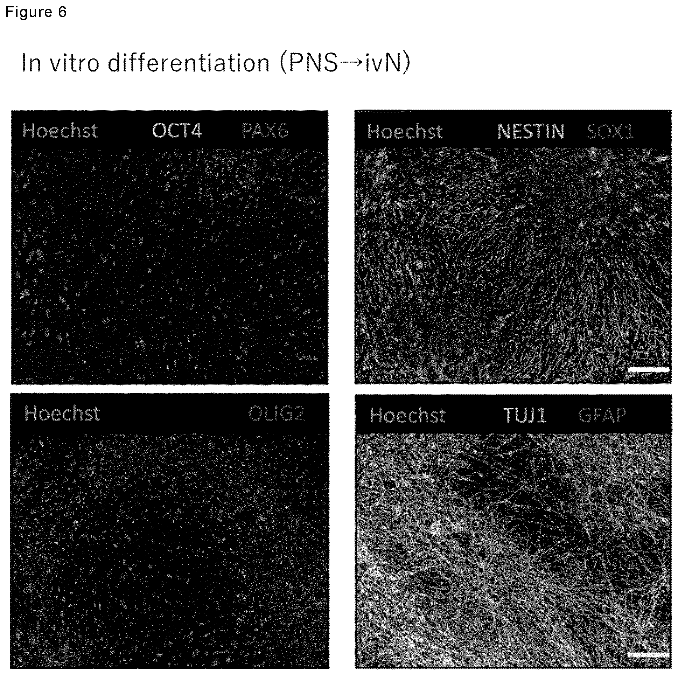

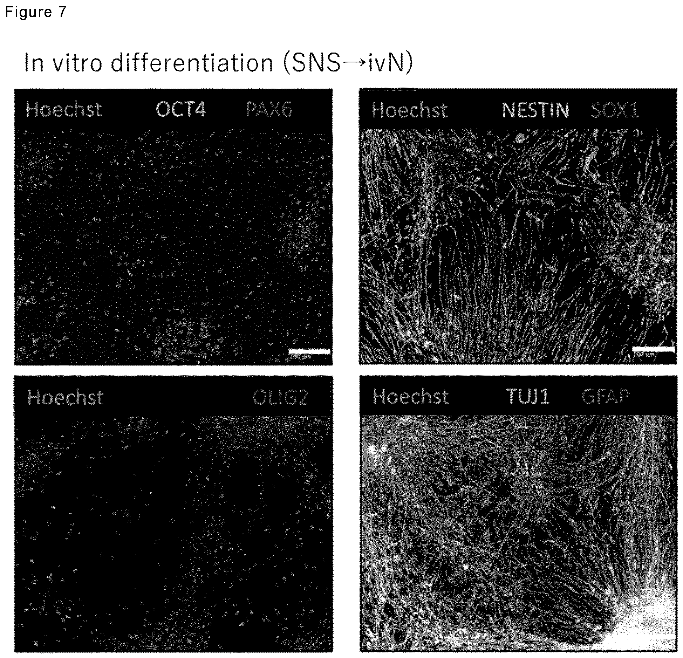

Next, the results on cell immunostaining in the case of differentiation induced *in vitro* from PNS are shown in Figure 6, and the results on cell immunostaining in the case of differentiation induced from SNS are shown in Figure 7, respectively. These results showed as follows;
· when PNS-derived cells were induced to differentiate *in vitro,* OCT4-positive cells were not found at all (Figure 6, top left), PAX6-, NESTIN- and SOX1-positive cells were found (Figure 6, top left, top right), OLIG2-positive cells were found (Figure 6, bottom left), TUJ1-positive cells were found (Figure 6, bottom right), and GFAP-positive cells were found (Figure 6, bottom right). These results suggested that though the cells composing PNS do not contain undifferentiated iPS cells, and a part of which maintains the state of neural stem cells/neural progenitor cells, the cells have the ability to differentiate into 3 cell lineages of neurons, astrocytes and oligodendrocytes.
· when SNS-derived cells are induced to differentiate *in vitro,* OCT4-positive cells were not found at all (Figure 7, top left), PAX6-, NESTIN- and SOX1-positive cells were found (Figure 7, top left, top right), OLIG2-positive cells were found (Figure 7, bottom left), TUJ1-positive cells were found (Figure 7, bottom right), and GFAP-positive cells were found (Figure 7, bottom right). These results suggested that though the cells composing SNS do not contain undifferentiated iPS cells, and a part of which maintains the state of neural stem cells/neural progenitor cells, the cells have the ability to differentiate into 3 cell lineages of neurons, astrocytes and oligodendrocytes. The above results showed that the PNS and the SNS have the similar differentiation potential.

### Example 8: Differentiation induction in vivo

The present example was carried out for the purpose of examining the therapeutic effect of the secondary neurospheres produced in Example 6 on a spinal cord injury *in vivo.*

In the present example, 8-week-old NOD-SCID mice were used. A spinal cord injury was induced in these mice by resecting the 10th thoracic vertebral arch and applying a pressure of 70 kdyn to the spinal cord using a specialized impactor equipped with a pressure sensor. On day 9 after the spinal cord injury, the SNS produced in Example 6 was transplanted. That is, 2 µ(micro)l of a solution containing 2.5 x 10⁵/µ(micro)l of SNS was injected taking over 2 minutes to the center part of spinal cord injury of the mouse that 9 days later from the injury surgery and motor dysfunction was confirmed to have been caused motor dysfunction 9 days later from the injury surgery (SNS; transplantation group). As a control, mice to which 2 µ(micro)l of only PBS was injected were used (PBS; non-transplantation group).

The movement of hind limbs during walking was evaluated using Basso Mouse Scale (BMS), respectively. BMS is the evaluation criteria which scores the movement of hind limbs during walking of the mouse spinal cord injury models on 9-point scale, and the specific evaluations were as described below. The scores of transplanted group; SNS (n=10) and non-transplanted group; PBS (n=11) were calculated as the mean +/-standard error (SEM), and statistical analysis was carried out by Repeated measure ANOVA, with p < 0.05 being considered to have a significant difference.
0 Point: No ankle movement
1 Point: Slight ankle movement
2 Point: Extensive ankle movement
3 Point: Plantar placing of the paw / Dorsal stepping
4 Point: Occasional plantar stepping
5 Point: Frequent or consistent plantar stepping, no coordination with forelimbs
6 Point: Frequent or consistent plantar stepping, some coordination with forelimbs
7 Point: Frequent or consistent plantar stepping, mostly coordinated with forelimbs, and severe trunk instability
8 Point: Frequent or consistent plantar stepping, mostly coordinated with forelimbs, and mild trunk instability /
9 Point: Normal

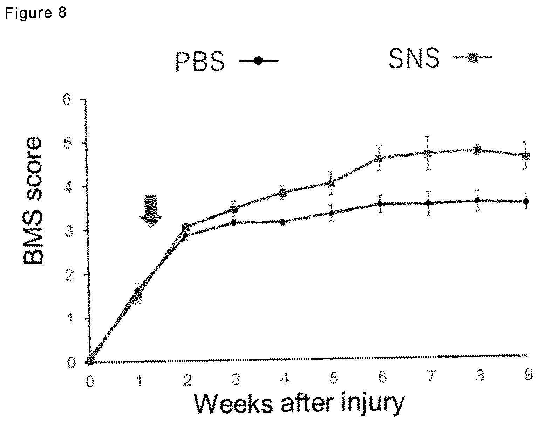

Figure 8 shows the results of the evaluation of the walking condition of the mice by the BMS score over time up to 9 weeks after the spinal cord injury. In this figure, SNS cells were transplanted or PBS was injected at the point of time shown with the downward arrow (day 9 after the spinal cord injury). The SNS cells used herein for transplantation were prepared by the same method as the production method of SNS cells of Example 7, except that, in the preparation method of SNS cells of Example 7, only during the last 24 hours in the last 7-days of culture of SNS production, the culture was carried out in the medium containing DAPT (10 µ(micro)M) (Selleck) of the γ(gamma)-secretase inhibitor as the Notch signaling inhibitor. Starting from about 2 weeks after the SNS transplantation, the improvement tendency in walking was found, and the BMS scores of SNS transplantation group and PBS injection group were 4.6 +/- 0.3, 3.5 +/- 0.2 (p < 0.01, Repeated measure ANOVA), respectively, thereby confirming the significant recovery of motor dysfunction.

Next, cell types of the *in vivo* differentiated transplanted cells were determined by visualizing each of the expressions of marker proteins by the fluorescent labeling using an antibody against the respective marker proteins of cell types.

After nine weeks from the spinal cord injury, a frozen sections of spinal cord from an individual mouse after the walking condition of the mouse was evaluated over time by BMS scores were air-dried and washed 3 times with PBS filled in a staining tray. Then, the sections were blocked using Blocking One (Nacalai Tesque Inc.) at room temperature for 20 minutes. Next, primary antibodies (anti-HNA antibody (Millipore), anti-OCT4 antibody (Millipore), anti-PAX6 antibody (Abcam), anti-HuC/D antibody (Invitrogen), anti-OLIG2 antibody (R&D), and anti-GFAP antibody (Synaptic system)), all diluted with Blocking One, were added, and then stored overnight at 4°C. Herein, HNA is a human nucleus marker; OCT4 is a iPS cell marker; PAX6 is the neural stem cell marker; HuC/D is a neuronal marker; OLIG2 is an oligodendrocyte marker; and GFAP is an astrocyte marker.

On the next day, the sections were washed 3 times with PBS, and secondary antibodies (donkey anti-rabbit IgG (Alexa Flour 555), donkey anti-mouse IgG (Alex Flour 488, Alex Flour 555), donkey anti-goat IgG (Alex Flour 555), goat anti-rat IgG (Alex Flour 488), goat anti-guinea pig IgG (Alex Flour 555), and Hoechst 33342 (all are Thermo Fisher Scientific Inc.)), all diluted with Blocking One, were added to react at room temperature for 1 hour. The sections were washed with PBS 3 times, Fluoromount (Diagnostic BioSystems) was added dropwise, a cover slip was placed, and the sections were allowed to stand at room temperature for 30 minutes to dry Fluoromount. Stained images were observed using a fluorescence microscope (Keyence).

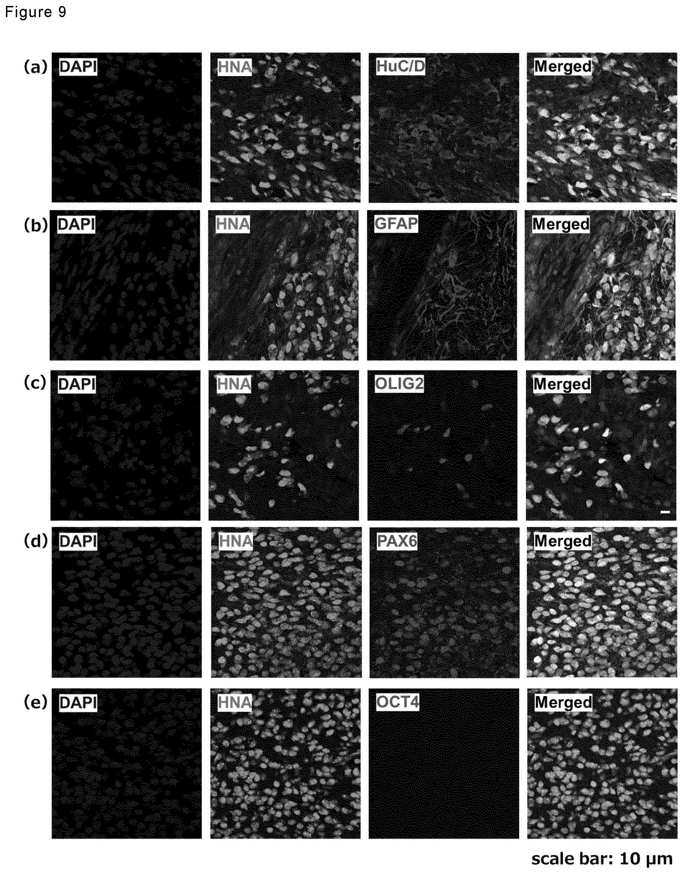

The results of fluorescent staining are shown in Figure 9. The cells positive for HNA, which is the human nucleus marker, shows transplanted cells. It was shown that many of the HNA-positive cells expressed HuC/D, which is the neuronal marker (Figure 9(a)), and some cells expressed GFAP, which is the astrocyte marker, and OLIG2, which is the oligodendrocyte marker (Figure 9(b) and (c)). Additionally, it was also confirmed that some HNA-positive cells expressed PAX6, which is the neural stem cell marker (Figure 9(d)). As shown in this figure, it was revealed that SNS transplanted in the spinal cord injury site mainly differentiated into neurons, and a part thereof differentiated into astrocytes and oligodendrocytes, and some cells maintained the state of neural stem cells. On the other hand, iPS cells showing OCT4 positive did not at all remain (Figure 9(e)).

### Industrial Applicability

According to the method of the present invention, by the step of culturing pluripotent stem cells for 5 days in a medium containing a SMAD signaling pathway inhibitor, a low concentration RA or RA analog, and an FGF signaling pathway inhibitor, it is possible to induce the pluripotent stem cells into neural stem cells/neural progenitor cells stably and with high reproducibility, and to improve more significantly the efficiency of the subsequent differentiation into neural lineage cells than conventional methods.

## Claims

1. A method for producing a neural stem cell/a neural progenitor cell from a pluripotent stem cell, comprising: a step of culturing a pluripotent stem cell for 5 days in a medium containing a SMAD signaling pathway inhibitor, retinoic acid (RA) or a RA analog at a concentration of 100 nM or less, and an FGF signaling pathway inhibitor.

2. The method according to claim 1, wherein the neural stem cell/the neural progenitor cell is **characterized by** the increased expressions of PAX6, SOX1 and NESTIN and by the suppressed expression of OCT4.

3. The method according to claim 1, wherein the pluripotent stem cell is selected from the group consisting of an ES cell and an iPS cell.

4. The method according to claim 2, wherein the pluripotent stem cell is selected from the group consisting of an ES cell and an iPS cell.

5. The method according to any of claims 1 to 4, wherein the SMAD signaling pathway inhibitor is SB431542 and LDN193189.

6. The method according to any of claims 1 to 4, wherein the RA analog is EC23.

7. The method according to any of claims 1 to 4, wherein the FGF signaling pathway inhibitor is SU5402.

8. The method according to any of claims 1 to 4, wherein the medium is selected from the group consisting of a medium for primate ES/iPS cells, AK02N medium, ciKIC (Registered trademark) iPS medium, mTeSR medium, NutriSTEM medium, and Essential 8 medium.

9. A method for producing a neurosphere from a pluripotent stem cell, the method comprising the steps of:
(i) culturing a pluripotent stem cell for 5 days in a medium containing a SMAD signaling pathway inhibitor, RA or a RA analog at a concentration of 100 nM or less, and an FGF signaling pathway inhibitor to produce a neural stem cell/a neural progenitor cell from the pluripotent stem cell, and
(ii) producing a neurosphere formed from the neural stem cell/the neural progenitor cell.

10. The method according to claim 9, wherein the step (ii) is carried out by culturing for 13 days in the presence of B-27 supplement (Registered trademark), Y27632, LIF, FGF, and RA or a RA analog.

11. The method according to claim 10, wherein the RA analog is EC23.

12. The method according to any of claims 9 to 11, wherein the step (ii) is carried out under a hypoxic condition.

13. The method according to any of claims 9 to 11, wherein a Notch signaling inhibitor is further added after 12 days from the start of the culture in the step (ii).

14. The method according to claim 13, wherein the Notch signaling inhibitor is a y(gamma)-secretase inhibitor.

15. The method according to any of claims 9 to 11, wherein after the step (ii), a step of dispersed the formed neurosphere into individual cells to produce a neurosphere again is further carried out once or more.

16. A method for producing a neural lineage cell from a pluripotent stem cell, the method comprising the steps of:
(i) culturing a pluripotent stem cell for 5 days in a medium containing a SMAD signaling pathway inhibitor, RA or a RA analog at a concentration of 100 nM or less, and an FGF signaling pathway inhibitor to produce a neural stem cell/a neural progenitor cell from a pluripotent stem cell,
(ii) producing a neurosphere formed from the neural stem cell/the neural progenitor cell, and
(iii) producing a neural lineage cell from the neurosphere.
